# FASCICULE DE BREVET EUROPEEN

(11) **EP 3 298 435 B1**
(45) Date de publication et mention de la délivrance du brevet: **27.07.2022**
(21) Numéro de dépôt: 16726366.4
(22) Date de dépôt: 13.05.2016
(51) Int. Cl.: G01T 1/164, G01T 1/20

(54) **SYSTEME ET PROCEDE DE DETECTION DE RAYONNEMENT GAMMA DE TYPE CAMERA COMPTON**
COMPTON-KAMERA-SYSTEM UND -VERFAHREN ZUR DETEKTION VON GAMMA-STRAHLUNG
COMPTON CAMERA SYSTEM AND METHOD FOR DETECTING GAMMA RADIATION

(30) Priorité: 18.05.2015 FR 1554435
(43) Date de publication de la demande: 28.03.2018
(73) Titulaire: Iltis, Alain, 10000 Troyes (FR)
(72) Inventeur: SNOUSSI, Hichem, 10450 Breviandes (FR)
(74) Mandataire: Debay, Damien
(86) Numéro de dépôt international: PCT/FR2016/051150
(87) Numéro de publication internationale: WO 2016/185123

(56) Documents cités:
- FR-A1- 2 997 766
- US-A1- 2002 008 205
- US-A1- 2002 011 571

## Description

### DOMAINE TECHNIQUE DE L'INVENTION

L'invention se rapporte à l'imagerie de sources de rayons gamma. Plus particulièrement, l'invention concerne un système de détection de rayons gamma de type caméra Compton permettant de reconstruire une image des sources gamma, de mesurer précisément les coordonnées spatio-temporelles et leurs énergies lorsque lesdits photons subissent une déviation Compton. L'invention concerne en outre l'utilisation du système de détection dans les domaines notamment de l'astronomie, de l'industrie nucléaire et du médical. L'invention concerne également le traitement de l'effet Compton dans les caméras temporelles.

### ETAT DE LA TECHNIQUE ANTERIEURE

A l'heure actuelle, l'imagerie de sources de rayons gamma (>30 KeV) s'effectue essentiellement à des fins de diagnostic médical autour de deux techniques : Le PET et le SPECT. Le SPECT est basée sur la scintigraphie et permet de réaliser des images et des reconstructions en trois dimensions des organes et de leur métabolisme au moyen d'un ensemble de gamma caméras tournant autour d'un patient. Le SPECT peut utiliser plusieurs énergies de rayons gamma, mais le collimateur en plomb qui permet de connaître leur direction d'arrivée en absorbe plus de 99%. Le PET utilise un anneau de détecteurs segmentés. Pour le PET on utilise des composés radio-pharmaceutiques émetteurs de positron. Ceux-ci donnent naissance à une paire de photons de 511 KeV dont il est possible de localiser l'émission grâce à leur détection simultanée sur l'anneau de détecteurs. Cependant les radio-eléments utilisés pour le PET ont une vie brève et sont donc coûteux.

Une troisième technique, la caméra Compton, est actuellement en émergence. Cette technologie permet comme le SPECT de faire une image quelle que soit l'énergie du gamma, mais contrairement au SPECT, tous les photons peuvent contribuer à l'image. Mais, les applications de la caméra Compton sont aujourd'hui limitées par son cout, le niveau élevé de bruit et la difficulté d'obtenir des reconstructions précises.

Lorsqu'on utilise des cristaux scintillants pour réaliser une image de sources de rayonnement gamma, on se heurte à la nature probabiliste de l'interaction photon gamma/matière. Nous remarquons essentiellement deux effets : premièrement le photon gamma peut être absorbé à une profondeur quelconque sur son trajet de propagation (Effet Depth of Interaction). Le deuxième effet consiste en ce que tous les systèmes d'imagerie actuels (matrice de pixels ou caméra d'Anger) reposent sur le postulat que l'endroit où a lieu le maximum d'émission de lumière est le lieu où le photon gamma a été détecté.

Du fait de la déviation Compton, ce postulat est juste sur la moyenne d'un grand nombre d'événements. Par contre, dans le cas d'un scanner de type PET si on reconstitue la position d'un événement unique, l'erreur, sur la position, peut être de plusieurs millimètres. La solution adoptée est alors de rejeter les événements pour lesquels l'énergie déposée n'est pas correcte. Cela conduit à rejeter un grand nombre d'événements.

L'objectif est de présenter une méthode pour traiter la diffusion Compton dans un nouveau type de détecteur la « caméra temporelle » décrite dans les demandes de brevets françaises numéro : FR2997766 A1 et WO2015173530 A2 du même déposant, et de décrire ainsi le fonctionnement d'une caméra Compton basée sur ce type de détecteur « caméra temporelle ».

Les techniques de traitement de la diffusion Compton ont eu jusqu'à aujourd'hui un succès limité car les caméras Compton pour fonctionner ont besoin d'une localisation précise d'au moins deux événements localisés en deux endroits distincts par exemple sur au moins deux plaques (plaque 1 & plaque 2) et d'une mesure précise de l'énergie déposée à chaque endroit sur chaque plaque. Pour cette raison, à ce jour toutes les caméras Compton fonctionnelles ont été réalisées avec des semi-conducteurs.

Les caméras Compton réalisées avec des semi-conducteurs présentent les problèmes suivants : Premièrement le pouvoir d'arrêt des cristaux semi-conducteurs est faible. Il faut donc des épaisseurs importantes supérieures à 30 mm pour absorber >80% du rayonnement à 51 1 KeV. Ces cristaux doivent être segmentés, chaque pixel étant lu séparément ce qui accroît le coût du système.

Le deuxième problème consiste en ce que le cout du cristal fonctionnalisé au cm<3>est élevé (environ 2000$/cm3) ce qui limite les caméras de petits systèmes.

Un autre problème des caméras Compton ainsi réalisées est que la réponse temporelle des semi-conducteurs est lente, supérieure à 10 ns. Or, beaucoup d'événements parasites sont mesurés pendant la mesure d'un événement Compton ce qui signifie qu'il y a beaucoup de bruit.

L'objet de l'invention est donc de proposer une solution technique permettant une détermination précise des coordonnées (X, Y, Z, T, E) de chaque événement gamma dans le cas où le photon incident a subi une diffusion Compton en utilisant les principes de la caméra temporelle.

L'invention propose ainsi un détecteur de type caméra Compton présentant les avantages suivants :
- C'est un détecteur de type spectromètre et imageur. Il permet donc de mesurer à la fois l'énergie des photons gamma et leur répartition spatiale.
- Le détecteur fonctionne pour une énergie quelconque du photon gamma, contrairement à la technique PET. Même si ce concept fonctionne d'autant mieux que l'énergie est élevée du fait de la focalisation du dépôt d'énergie dans la direction de propagation du rayon gamma.
- Le détecteur peut utiliser tous les photons gamma incidents du fait de l'absence de collimateur, contrairement au SPECT.

L'objet principal de l'invention est donc de proposer une nouvelle technique permettant - de conserver une bonne localisation de chaque événement dans un détecteur de type caméra temporelle dans le cas où le photon a subi un effet Compton ; - de mesurer l'énergie d'un événement dans un détecteur de type caméra temporelle dans le cas où le photon a subi un effet Compton ; et - de réaliser une caméra Compton améliorée en combinant un ou plusieurs détecteurs de type caméra temporelle.

### EXPOSE DE L'INVENTION

L'invention concerne un système selon l'une des revendications 1 et 2. Les revendications 3 à 11 détaillent divers particularités et avantages de divers modes de réalisation.

L'invention concerne également un procédé selon l'une des revendications 12 ou 13 et une utilisation selon la revendication 14.

### BREVE DESCRIPTION DES FIGURES

D'autres caractéristiques, détails et avantages de l'invention ressortiront à la lecture de la description qui suit, en référence aux figures annexées, qui illustrent :
la figure 1 montre le système de détection selon l'invention dans le cas d'une plaque P1 et lorsque le photon ayant subi une diffusion Compton d'angle α_{c} reste dans un même cône de lumière (α_{c} inférieure à θ_{c}) ;
la figure 2 montre le système de détection selon l'invention dans le cas d'une plaque P1 lorsque le photon ayant subi une diffusion Compton d'angle α_{c} sort du cône de lumière (déviation α_{c} supérieure à θ_{c}) existence de deux cercles distincts ;
la figure 3 montre le système de détection selon l'invention dans le cas où (déviation α_{c} supérieure à θ_{c}) existence de deux cercles confondus, la distribution de lumière étant ajustée par une ellipse ;
la figure 4 montre le principe de la figure 3, existence de deux cercles confondus, la distribution de lumière étant ajustée par une composition de deux cercles ;
la figure 5 montre le système de détection selon l'invention avec la plaque P1 muni de deux réseaux de photo-détecteurs respectivement sur une face d'entrée et une face de sortie ;
la figure 6 montre un mode de réalisation du système avec deux plaques de scintillateurs P1 et P2.

### DESCRIPTION DETAILLEE

La présente invention utilise une caméra temporelle capable de mesurer à la fois la position dans l'espace, dans le temps ainsi que l'énergie de chaque photon gamma. Le principe d'une caméra temporelle est enseigné dans les demandes de brevets FR2997766 A1 et WO2015173530 A2 du même déposant.

Dans ce type de caméra temporelle, pour chaque événement de scintillation (effet photo-électrique ou diffusion Compton) on identifie un cercle correspondant aux photons non diffusés qui sont les premiers détectés.

Les photons non diffusés sont répartis dans un cône dont le sommet est le lieu de l'interaction (X, Y, Z, T) et dont l'angle d'ouverture est l'angle de réflexion totale sur la face de sortie.

Lorsque le photon gamma subit un effet photo-électrique, on n'a qu'un seul cercle. Dans tout ce qui suit on cherche à caractériser la position et le diamètre d'un cercle et non le barycentre d'une distribution de lumière.

Si le photon gamma subit une diffusion Compton puis un effet photoélectrique, on a deux cercles qui apparaissent de façon quasi coïncidente sur le plan des détecteurs. Un bon critère expérimentalement pour valider la présence ou non d'une diffusion Compton est que la différence entre les temps d'arrivée Ta-Tb des deux maximums de lumière doit être inférieure à trois fois le temps de transfert de la lumière dans le cristal. [0037] Dans le cas où le photon gamma subit une déviation Compton au point A (Xa, Ya, Za, Ta, Ea) avant d'être absorbé au point B (Xb, Yb, Zb, Tb, Eb), on considérera les trois cas suivants :

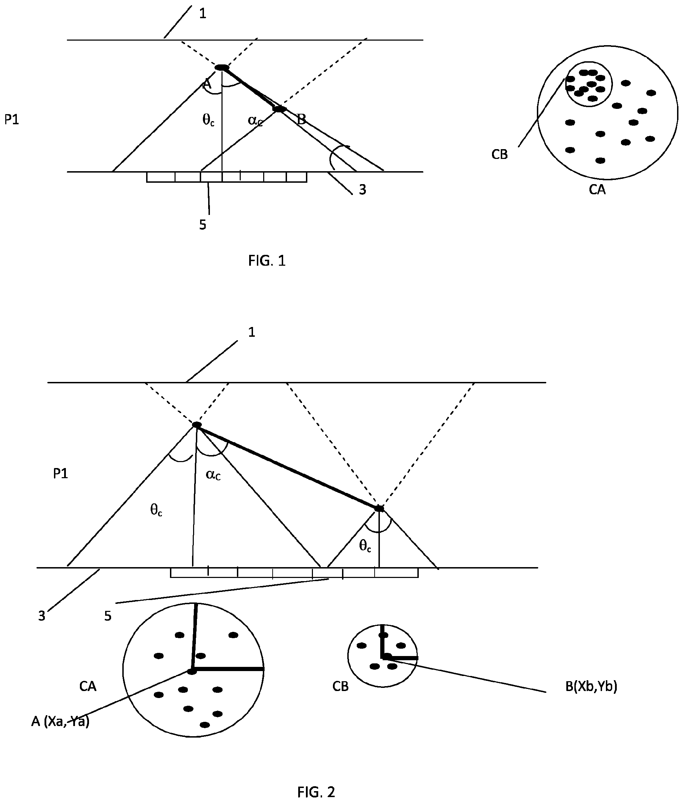

La figure 1, montre le système de détection selon l'invention dans un premier cas où le photon subit une diffusion Compton d'angle α_{c} restant dans un même cône de lumière que les photons non diffusés. Le système comprend une plaque P1, muni d'un réseau de photo-détecteurs 5 associé à des composants micro-électroniques 6. La plaque P1 est d'une épaisseur supérieure ou égale à 10 mm. Sur cette plaque P1 on détecte le temps Ta d'arrivée des photons non diffusés émis par une diffusion Compton en un premier point A ; ensuite on détecte le temps Tb d'arrivée des photons ayant subi une absorption totale en un second point B ; et on détermine un cercle C_{A} correspondant aux photons émis lors d'une déviation Compton ainsi qu'un cercle C_{B} correspondant aux photons émis lors de l'absorption complète du photon gamma. Dans ce premier cas, la déviation Compton d'angle α_{c} est inférieure à l'angle θ_{c} où θ_{c} est l'angle critique de réflexion totale. Dans ce cas qui est le plus courant, les photons non diffusés émis par l'interaction restent tous dans le même cône de lumière, mais leur répartition peut présenter une asymétrie i.e. le cercle C_{B} (correspondant au point B) inclus dans le cercle C_{A} correspondant au point A. Or, de la même manière que pour un événement photo-electrique le diamètre du cercle C_{A} permet de mesurer Xa, Ya et Za. Ainsi, le diamètre du cercle C_{B} permet de mesurer Xb, Yb et Zb. La reconstitution de l'interaction permet de mesurer Ta et Tb. L'énumération du nombre de photons dans les cercles C_{A} et C_{B} permet d'estimer l'énergie relative déposée aux points A et B. Dans une version simplifiée du traitement on ne considère que le cercle le plus large (cercle C_{A}). On remarque que l'effet Compton ne modifie pas la précision de la mesure des coordonnées spatiotemporelles du point A. On considère aussi, comme illustré sur la figure 2, le cas où le photon ayant subi une diffusion Compton d'angle α_{c} sort du cône de lumière. Dans ce cas la déviation α_{c} est supérieure à θ_{c}. On obtient des événements distincts, c'est-à-dire existence de deux cercles quasi simultanés dans le temps, contrairement au cas du pile-up. Là encore on a deux cas : soit les deux cercles sont confondus (Distance A B < Ra + Rb), soit les deux cercles sont distincts (Distance A- B > Ra + Rb). La figure 2 représente le cas où les deux cercles C_{A} et C_{B} sont distincts. On reconnaît qu'il s'agit d'une diffusion Compton si Tb - Ta est voisin de Distance (A-B)/C. Dans ce cas on traite chaque événement indépendamment. Le premier événement détecté et/ou selon la géométrie celui correspondant à l'énergie déposée la plus importante est l'événement initial. L'énergie du photon initial est égale à EA +EB. La position des deux points permet de mesurer α_{c}.

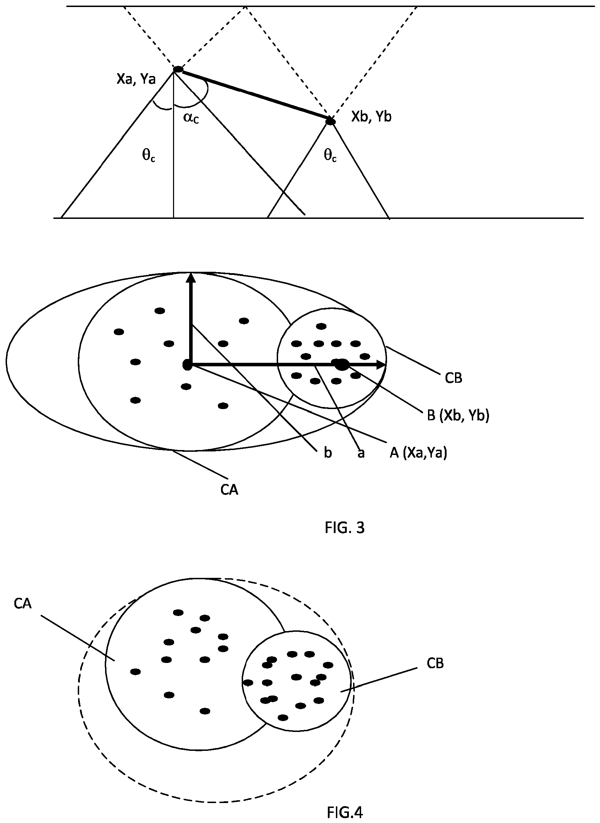

Dans le cas où les cercles C_{A} et C_{B} sont confondus, on reconnaît qu'il s'agit d'une diffusion Compton si Tb-Ta est voisin de A-B/C. Dans ce cas comme représente sur la figure 3 on peut ajuster la distribution de lumière par une ellipse dont le demi petit axe (b) correspond au rayon du premier cercle RA et de demi grand axe (a) correspond à Distance A-B + RB. Dans cette configuration, on conserve l'essentiel de la précision dans l'estimation des coordonnées Xa, Ya et Za malgré la diffusion Compton. La position du point A (Xa, Ya) est donnée par le centre de l'ellipse. La profondeur d'interaction en A est donnée par le demi petit axe de l'ellipse, ce qui permet de calculer Za. On peut ensuite calculer Ta en corrigeant les temps mesurés avec Za. Ainsi la position du point B est donnée par le foyer de l'ellipse (Xb, Yb). La profondeur d'interaction en B est donnée par le demi grand axe de l'ellipse (A-B +RB). Connaissant A-B, on trouve RB, ce qui permet de calculer Zb. On peut ensuite calculer Tb en corrigeant les temps mesurés avec Zb. L'énergie E est mesurée en intégrant les photons sur l'ensemble de ladite ellipse. On obtient EA+EB l'énergie totale. En calculant le barycentre de la distribution des photons dans l'ellipse on peut trouver le point initial d'interactions A ou B qui est le plus proche du barycentre.

Une autre méthode dans le cas où les deux cercles C_{A} et C_{B} sont confondus est d'ajuster la distribution de lumière par une composition de deux cercles telle que représenté sur la figure 4 en trait pointillé. Dans ce cas le centre de chaque cercle donne la position des interactions respectives Xa, Ya en A et Xb, Yb en B. Les diamètres des cercles C_{A} et C_{B} donnent la profondeur d'interaction Za et Zb. L'énergie est mesurée en intégrant les photons sur l'ensemble de ladite composition. Le point initial d'interaction A ou B est celui qui est le plus proche du barycentre de la distribution globale de lumière. Par ailleurs, la reconstitution de la position des deux interactions permet d'estimer l'angle de déviation Compton α_{c}. Le ratio EB/EA doit alors vérifier les lois de la diffusion Compton.

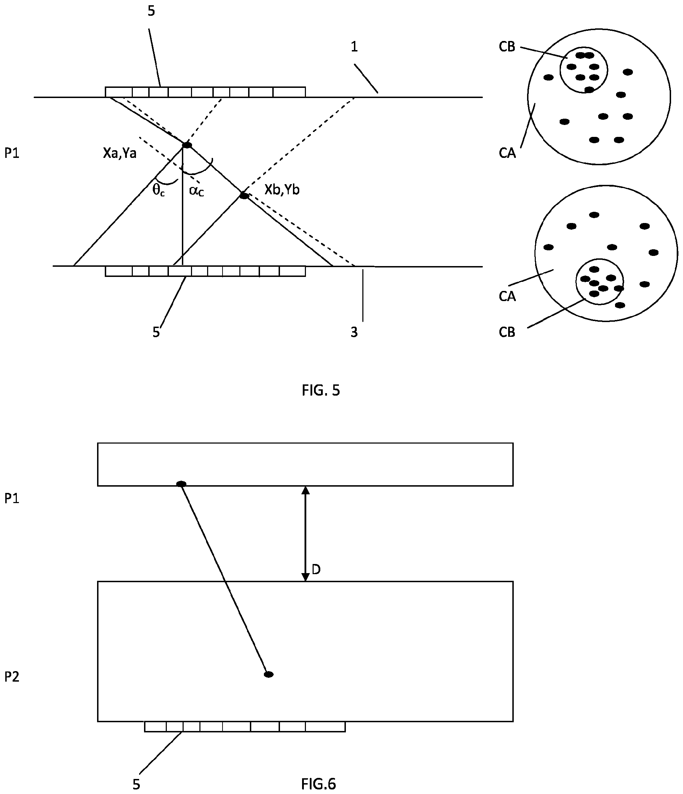

La figure 5 représente le même cas que la figure 1 sauf que la face d'entrée 1 et la face de sortie 3 de la plaque P1 sont recouvertes de photo-détecteurs segmentés avec une densité de remplissage maximum. Dans ce cas, les photons qui ont subi une déviation Compton d'angle de déviation αc sont absorbés par le photo- détecteur. On voit le cercle des photons non diffusés sur chaque côté de la plaque et on réalise exactement les mêmes traitements décrits précédemment. Cependant la propagation du rayon gamma de la face d'entrée 1 vers la face de sortie 3 introduit une asymétrie entre les deux faces. D'une certaine manière l'image sur la face d'entrée 1 est l'inverse de l'image sur la face de sortie 3.

L'avantage de recouvrir chaque côté de la plaque P1 de photo-détecteurs 5 est qu'on obtient deux estimations indépendantes des coordonnées de chaque événement (X, Y, Z, T, E). De plus, la comparaison des distributions de lumière de chaque côté de la plaque permet de lever d'éventuelles ambiguïtés dans la reconstitution si le photon a subi au moins une diffusion Compton. Par ailleurs, on double le nombre de photons utilisés pour la reconstruction et ainsi on améliore la résolution en énergie pour chaque photon car la résolution en énergie s'accroit avec le nombre de photons collectés.

L'inconvénient de la configuration ci-dessus se trouve dans le coût des composants micro-électronique utilisés sur chaque plan de photo-détecteurs. En effet, dans ce cas on double le prix de l'électronique. Cette configuration est intéressante essentiellement sur une plaque épaisse et surtout dans le cas d'une caméra Compton monoplaque où l'on cherche à reconstituer le trajet du photon gamma dans le cristal. Par ailleurs cette configuration ne permet pas de rejeter le bruit qu'il soit intrinsèque au scintillateur ou non. Tous les événements sont analysés qu'ils proviennent de la source ou non.

Pour des photons d'énergie connue (PET) ou de direction d'arrivée connue (SPECT), on peut simplifier le dépouillement des événements. Dans ce cas en effet, on cherche uniquement à déterminer le point d'impact initial du photon (XA, YA, ZA), son temps d'arrivée TA et l'énergie globale de l'interaction EA+EB. Dans ce cas la double lecture est avantageuse.

Nous avons vu que dans tous les cas il est possible de mesurer correctement la position de l'interaction initiale malgré la diffusion Compton. En outre, il est possible d'avoir une estimation du vecteur AB entre les deux interactions successives et de l'énergie EA et EB déposée à chaque interaction. Il est donc possible avec un tel système de réaliser une caméra Compton avec une seule plaque de scintillateur. Un système monoplaque ne sera pas optimum en termes de performance, mais il sera très avantageux en termes de coût et en termes d'efficacité de détection (fort pourcentage des rayons gamma totalement absorbés par le détecteur). Si on utilise la caméra Compton dans un environnement bruyant tel que des plaques détectrices en LSO possédant un fort rayonnement intrinsèque et un fort débit de rayonnement, il est nécessaire d'utiliser un système à deux plaques. En effet, la coïncidence temporelle entre la détection sur les deux plaques est un excellent moyen pour contrer le bruit. Un événement Compton valide doit être détecté quasi simultanément sur les deux plaques. Le retard entre 2 événements valides ne peut pas être supérieur à Temps transfert de plaque P1 (c/n) + Temps de transfert inter-plaques (c) + Temps de transfert plaque P2 (c/n) soit un temps <1 ns.

Dans un autre mode de réalisation du système afin de supprimer la réflexion des rayons gamma sur les faces non utilisées pour la détection (faces latérales et face d'entrée si on n'a qu'un seul réseau de détecteurs), celles-ci sont traitées de telle manière que l'absorption des photons incidents y soit maximum. En effet, si les photons sont réfléchis sur ces faces, ils viennent bruiter la détection du cercle des non diffusés. Le fait de traiter toutes les faces non utilisées pour la détection, faces dites « stériles », permet d'augmenter de 50 à 100% le temps d'intégration des images temporelles (i.e. de les faire passer de 750 ps à 1500 ps) pour un taux de photons non diffusés détectés donné (i.e. 90% des photons détectés sont non diffusés). Ce mode n'est possible que si on mesure l'énergie par la méthode temporelle, c'est à dire pour des énergies > 250 KeV dans le LSO. Les faces non couplées à un réseau de détecteurs sont rugueuses et traitées de manière à absorber autant que faire se peut le rayonnement incident afin d'éviter les réflexions parasites vers les détecteurs.

Ce traitement doit éviter les reflets sur les faces dites « stériles », notamment par saut d'indice. Le traitement peut être composé d'un dépôt Anti-reflet de tout type connu, suivi du dépôt d'une couche de matériau absorbant. Il peut également être constitué d'un dépôt d'une résine à haut indice (n>1 ,5) chargée en matériau absorbant.

Si les faces « stériles » sont simplement peintes en noir, de manière classique du fait du contraste d'indice important entre le cristal (n= 1 ,9) et la peinture (n<1 ,5) une grande partie des photons est réfléchie vers l'intérieur du cristal.

Les faces couplées à des réseaux de détecteurs sont préférentiellement polies. Le couplage entre ces faces et les détecteurs est réalisé par un média d'indice faible (n<1 ,5) afin de ménager un angle de réflexion totale. [0050] Pour réaliser une caméra temporelle couvrant une large gamme d'énergie (100 KeV -2 MeV) il peut être avantageux qu'au moins une des deux plaques mesure l'énergie des photons par la méthode classique (traitement diffusant blanc)

Simulation des trois cas d'effet Compton Les conditions de la simulation sont les suivantes :
Cristal LaBr3 : Ce d'épaisseur 30 mm d'indice n= 1 ,9 couplage avec le photodétecteur avec de la graisse (n = 1 ,4). Pour chaque image on indique la position des photons détectés à un temps donné :
- 200 ps
- 700 ps
- 16000 ps (16ns)

Chaque image montre également une simulation de ce qu'on voit sur chaque photodétecteur segmenté.

Point A de la diffusion Compton positionnée à Z = 5 mm pour les trois cas :
Cas n (alpha < thêta) point B absorption totale à Z = 15 mm.
Cas n °2 (alpha >thêta) Compton adjacente : point B absorption totale à Z = 15 mm.
Cas n °3 (alpha = pi/2) Compton disjoint : point absorption totale à Z = 5 mm.

Dans les photo-détecteurs actuels, la détection des photons est sujette à des effets de seuil. Si on veut sortir du bruit de fond des détecteurs (dark counts) il est nécessaire de détecter 1,5 à 2 photo-électrons. Le temps d'intégration Ti étant bref, typiquement inférieur à 2 ns, le nombre de photons à détecter pendant Ti peut être inférieur au seuil pour les pixels périphériques. Le temps d'intégration est donné par le temps au bout duquel le nombre des photons détectés hors du cercle des photons non diffusés passe un certain seuil. Le nombre de photons émis par interaction dans le secteur angulaire des photons non diffusés est constant. La densité de photons/pixels dépend du diamètre du cercle. Le diamètre maximum du cercle dépend de l'épaisseur du cristal. On peut donc jouer sur la densité de photons/pixels en jouant sur l'épaisseur du scintillateur cristal. Ainsi, plus le détecteur sera finement segmenté, plus il pourra être avantageux d'utiliser des cristaux peu épais.

Par ailleurs le temps d'intégration Ti (inférieur à 2 ns) étant bref vis-à-vis des possibilités des meilleures électroniques actuelles, il est avantageux de chercher des moyens permettant de compter plus longtemps les photons. Le temps d'intégration donné par le temps au bout duquel le nombre des photons détectés hors du disque des photons non diffusés passe un certain seuil (par exemple 90%), le passage de ce seuil dépend essentiellement du nombre de photons diffusés sur la face d'entrée 1 du cristal, ou sur les faces latérales pour les pixels situés à moins d'une épaisseur de cristal des bords. Etant donné que pour un imageur Compton, on peut n'utiliser pour mesurer X, Y, Z, T, E que les photons non diffusés, il peut être avantageux de supprimer tous les autres photons.

Une manière connue de réaliser ceci peut être de peindre les faces latérales et la face d'entrée 1 (les faces non utilisées pour la détection) en noir afin d'absorber tous les photons qui sortent du cristal. Cependant étant donné que l'indice de la peinture (typiquement 1 ,5) est inférieur à l'indice du cristal 1 ,8 à 1 ,9, une majorité des photons sont réfléchis par le saut d'indice et reviennent perturber le signal. Une manière plus avantageuse de réaliser l'invention est donc soit de trouver un produit noir d'indice proche de celui du scintillateur, soit de réaliser un traitement anti-reflet par tout moyen connu sur les faces latérales et la face d'entrée 1 du cristal, puis d'appliquer un dépôt noir absorbant sur ce traitement anti-reflet.

Une autre manière de réaliser ce résultat peut être de déposer sur ces faces du cristal une résine d'indice « n » élevé (n>1 ,5) de préférence n supérieur à 1 ,7 chargée en particules absorbantes.

Ce traitement présente les avantages suivants : En diminuant fortement le nombre de photons détectés hors du cône de lumière des photons non diffusés, on augmente le temps pendant lequel on peut compter les premiers photons pour définir la position du cercle.

Ce système permet également de limiter fortement les effets de bord et donc d'exploiter l'intégralité du détecteur pour l'imagerie.

Ce traitement anti-reflet peut être réalisé par couches d'interférences, cristaux photoniques ou adaptation progressive d'indice obtenue par nanostructuration telle que divulgué dans la demande de brevet européen n ° 4305365.0 déposée le 13 mars 2014 « Structuration pour l'optimisation de la collection de photons dans les cristaux scintillateurs et solutions technologiques associées ».

La figure 6 représente un deuxième mode de réalisation du système selon l'invention comprenant deux plaques de scintillateurs P1 et P2, des réseaux de photo-détecteur 5 et l'électronique associée non représentée, les réseaux étant collés à chaque plaque P1 et P2. La plaque P1 est plus fine que la plaque P2. Sur cette plaque P1 on cherche à obtenir une diffusion Compton en un premier point A de coordonnées (Xa, Ya, Za, Ta, Ea). La seconde plaque P2 est plus épaisse que la plaque P1. L'épaisseur de ladite seconde plaque P2 permet d'absorber au moins 50% de l'énergie du rayon gamma en un point B de coordonnées (Xb, Yb, Zb, Tb, Eb). La seconde plaque P2 est séparée de la plaque P1 par une distance 'D' d'au moins 10 mm, de préférence 30 mm. Le système comporte un module d'estimation d'un événement Compton valide. Ledit module est apte à mesurer sur la seconde plaque P2 un trigger de coïncidence Tb dans une fenêtre temporelle inférieure à 1 ns pour identifier les événements Compton valides.

Soit D' la distance entre les 2 plaques de la caméra Compton, EP1 l'épaisseur de la première plaque, EP2 l'épaisseur de la deuxième plaque. Le temps de transfert maximum d'un photon perpendiculaire au détecteur est :

Le temps Tmax = EP1<*>(n/c) + D'/c + EP2<*>(n/c). Pour simplifier, dans le cas d'une propagation oblique, on considère T < 1 ,5 Tmax.

Les temps de détection d'un événement Compton détecté sur les deux plaques devra donc vérifier : D'/c < TB-TA < 1 ,5 Tmax. Dans le cas d'un système en LaBr3 optimisé pour 51 1 KeV (EP1 = 10 mm, D'= 30 mm, EP2 = 30 mm) cela donnerait : 100 ps <TB -TA < 380 ps.

Cette condition temporelle très stricte permet de rejeter tous les événements qui ne sont pas des diffusions Compton strictes. Un fenêtrage temporel aussi précis est possible avec les électroniques développées pour les caméras temporelles et pour les détecteurs de type Si-PM digital.

La probabilité de deux événements coïncidents dans un temps aussi court (hors Compton) est très faible. Ce fenêtrage permet donc une forte réduction du bruit des détecteurs.

On voit donc que l'invention permet de faire deux types de caméra Compton : 1) Une caméra monoplaque d'une précision et d'une sensibilité modérée mais compacte et d'un cout modéré. 2) Une caméra multiplaque très sensible du fait du rejet du bruit par le fenêtrage temporel, plus précise du fait de la meilleure définition angulaire du trajet du photon gamma, mais plus encombrante et chère.

On remarque que dans le système de détection selon l'invention constitué d'une seule plaque P1 ou de deux plaques P1 et P2, on conserve une bonne localisation de chaque événement dans un détecteur dans le cas où le photon a subi un effet Compton et de plus on mesure précisément l'énergie d'un événement dans un détecteur de type caméra temporelle dans le cas où le photon a subi un effet Compton.

Par ailleurs, on peut réaliser une caméra Compton améliorée en combinant un ou plusieurs détecteurs de type caméra temporelle.

Un autre intérêt du système selon invention, est son utilisation dans des domaines notamment médical et de l'astronomie. Le système de détection selon l'invention peut en outre être utilisé dans l'industrie pour détecter des contaminations radioactives.

De nombreuses combinaisons peuvent être envisagées sans sortir du cadre de l'invention, qui est définie par les revendications ; l'homme de métier choisira l'une ou l'autre en fonction des contraintes économiques, ergonomiques, dimensionnelles ou autres à respecter.

## Revendications

1. Système de détection de rayonnement gamma, de type caméra Compton, comprenant une source de rayonnement gamma, une plaque de scintillateur rapide P1 dont le temps de montée au pic de lumière est inférieur à 1ns, présentant une épaisseur supérieure ou égale à 5 mm, équipée d'un réseau de photodétecteurs segmentés (5) et d'une micro-électronique de lecture rapide dédiée, ledit système étant **caractérisé en ce qu'**il comporte un module d'estimation d'un événement Compton valide capable de mesurer les coordonnées spatio-temporelle (X, Y, Z, T) et l'énergie E en au moins deux positions successives d'un photon gamma lorsque ledit photon subit une déviation Compton en un premier point A à un temps TA avant d'être absorbé en un second point B à un temps TB, en reconnaissant des cercles de photons non diffusés correspondant à chaque interaction de scintillation, ledit module est apte à réaliser cette estimation d'un événement en identifiant au moins un premier et un second extremum dans la distribution de lumière à l'intérieur de ladite plaque P1, ledit second extremum apparaissant lorsque la différence entre un temps Ta d'arrivée des photons en un premier point A, et un temps Tb d'arrivée des photons en un second point B, est inférieure à trois fois d'un temps de transfert Tt de la lumière dans ladite plaque P1,et **en ce que** ledit système est apte à déterminer, d'une part, un cercle C_{A} correspondant aux photons non diffusés émis par la déviation Compton du rayonnement gamma au point A, le diamètre du cercle C_{A} permettant de mesurer Xa, Ya et Za, d'autre part, un cercle C_{B} correspondant aux photons non diffusés émis par l'absorption totale du photon gamma au point B, le diamètre du cercle C_{B} permettant de mesurer Xb, Yb, et Zb.

2. Système de détection de rayonnement gamma, de type caméra Compton, comprenant une source de rayonnement gamma, deux plaques (P1, P2) de scintillateur rapide dont le temps de montée au pic de lumière est inférieur à 1ns, la plaque P1 étant plus fine que la seconde plaque P2, l'épaisseur de la plaque de scintillateur P1 étant telle que le photon gamma subit une déviation Compton en un point A de ladite plaque P1, ladite seconde plaque P2 étant séparée de la plaque P1 par une distance 'D' d'au moins 10 mm, de préférence supérieure à l'épaisseur de la plaque la plus épaisse, un réseau de photodétecteurs segmentés et une micro-électronique de lecture rapide dédiée, ledit système étant **caractérisé en ce qu'**il comporte un module d'estimation capable de mesurer les coordonnées spatio-temporelle (X, Y, Z, T) et l'énergie E en au moins deux positions successives d'un photon gamma lorsque ledit photon subit une déviation Compton en un premier point A avant d'être absorbé en un second point B, en reconnaissant des cercles de photons non diffusés correspondant à chaque interaction de scintillation, ledit module est apte à mesurer sur ladite seconde plaque P2 un trigger de coïncidence dans une fenêtre temporelle inférieure au temps de transfert maximum de la lumière entre les plaques P1 et P2 pour identifier les événements Compton valides, ce trigger de coïncidence étant inférieur à 1 ns, et **en ce qu'**il est apte à déterminer, d'une part, un cercle C_{A} correspondant aux photons non diffusés émis par la déviation Compton du rayonnement gamma au point A, le diamètre du cercle C_{A} permettant de mesurer Xa, Ya et Za, d'autre part, un cercle C_{B} correspondant aux photons non diffusés émis par l'absorption totale du photon gamma au point B, le diamètre du cercle C_{B} permettant de mesurer Xb, Yb, et Zb.

3. Système de détection de rayonnement gamma de type caméra Compton selon la revendication 1 **caractérisé en ce qu'**il comprend une seule plaque de scintillateur P1 d'épaisseur supérieure ou égale au libre parcours moyen du rayon gamma dans le cristal considéré.

4. Système de détection de rayonnement gamma, de type caméra Compton selon la revendication 3 **caractérisé en ce qu'**il comprend deux réseaux de photo-détecteurs disposés respectivement sur une face d'entrée (1) et une face de sortie (3) de ladite plaque scintillateur P1 afin d'améliorer la précision de la reconstitution du trajet Compton dans la plaque de scintillateur P1.

5. Système de détection de rayonnement gamma, de type caméra Compton selon la revendication 4 dans lequel la face d'entrée (1) et la face de sortie (3) de la plaque scintillateurs (P1) étant couplées au réseau de photo-détecteurs (5) sont polies et le couplage entre lesdites faces et le réseau de photo-détecteurs est réalisé par un média d'indice n inférieur 1.5, afin de ménager un angle de réflexion totale.

6. Système de détection selon la revendication 1 **caractérisé en ce que** des faces latérales et une face d'entrée (1) de ladite plaque P1, n'étant pas couplées à un réseau de photo-détecteurs (5) sont rugueuses, lesdites faces sont traitées de sorte que l'absorption des photons incidents ou la réflexion diffuse des photons, y soit maximum.

7. Système de détection selon la revendication 6 dans lequel, la face d'entrée (1) de la plaque P1 non couplée à un réseau de photo-détecteur est peinte en noir afin de limiter la réflexion sur ladite face.

8. Système de détection selon la revendication 1 **caractérisé en ce que** des faces latérales et une face d'entrée (1) de ladite plaque (P1) n'étant pas couplées à un réseau de photo-détecteurs (5) sont enrobée d'un réflecteur blanc avec un gap d'air avec la plaque P1, de sorte qu'elles soient réfléchissantes et diffusante.

9. Système selon l'une des revendications précédentes **caractérisé en ce que** la mesure de l'énergie se fait de manière conventionnelle en collectant par réflexion diffuse un maximum de photons émis sur au moins l'une des deux plaques.

10. Système de détection selon l'une des revendications précédentes dans lequel le réseau de photo-détecteurs fragmenté (5) est de type SI-PM analogique associé avec un ASIC analogique ou de type SI-PM numérique.

11. Système de détection selon l'une des revendications précédentes **caractérisé en ce que** les plaques de scintillateurs P1 et P2 sont de type silicate de lutétium et/ou halogénure de lanthane.

12. Procédé de détermination des coordonnées spatio temporelles (X, Y, Z, T) et de l'énergie E en au moins deux positions successives d'un photon gamma ayant subi une diffusion Compton mis en œuvre dans un système selon l'une des revendications 1 à 11 comprenant les étapes suivantes :
- détection d'un temps Ta d'arrivée des photons non diffusés émis par la diffusion Compton en un premier point A;
- détection d'un temps Tb d'arrivée des photons non diffusés émis en un second point B par l'absorption totale du photon gamma;
- Détermination d'un cercle C_{A} correspondant aux photons non diffusés émis par la déviation Compton du rayonnement gamma au point A, le diamètre du cercle C_{A} permettant de mesurer Xa, Ya et Za;
- Détermination d'un cercle C_{B} correspondant aux photons non diffusés émis par l'absorption totale du photon gamma au point B, le diamètre du cercle C_{B} permettant de mesurer Xb, Yb et Zb ;
lorsque les photons émis lors de la diffusion Compton en A et de l'absorption totale en B restent dans un même cône de lumière des photons non diffusés émis en A, l'angle α_{C}< θ_{C} où α_{C} est la déviation Compton et θ_{c} est l'angle critique de réflexion totale et le cercle C_{B} est inclus dans le cercle C_{A}, dans ce premier cas, le procédé comprend en outre des étapes suivantes :
- Calcul des diamètres desdits cercles C_{A} et C_{B} afin de mesurer (Xa, Ya, Za) et (Xb, Yb, Zb) ;
- Enumération des nombres de photons dans lesdits cercles C_{A} et C_{B} inscrits;
- Définition de l'énergie d'un photon gamma, lesdites énergies Ea et Eb étant proportionnelle au nombre de photons comptés à l'intérieur desdits cercles C_{A} et C_{B} ; ou
lorsque le photon ayant subi une déviation Compton sort du cône de lumière, α_{C} > θ_{C}, la distance entre les points A et B est grande et les cercles C_{A} et C_{B} sont distincts dans cette seconde configuration le procédé comprend en outre:
- Détermination d'un premier événement A correspondant à l'énergie la plus importante;
- Mesure des coordonnées (Xa, Ya, Za, Ta) dudit événement en A et son énergie Ea ;
- Détermination d'un second événement B correspondant à l'énergie la plus faible ;
- Mesure des coordonnées de l'événement (Xb, Yb, Zb, Tb) et son énergie Eb;
- Mesure de l'énergie initiale du photon gamma équivalente à la somme des énergies Ea + Eb;
- Détermination d'un angle de déviation Compton en reconstituant la position des deux interactions ;
- Déduction de la direction d'arrivée du photon gamma, à partir de la position du point A (Xa, Ya, Za), de la position du point B (Xb, Yb, Zb) et des énergies Ea et Eb ; ou
lorsque le photon ayant subi une déviation Compton sort du cône de lumière α_{C} > θ_{c}, la distance entre les points A et B est petite et les cercles C_{A} et C_{B} sont confondus, dans ce troisième cas, le procédé peut comprendre les étapes suivantes:
- Ajustement de la distribution de lumière par une ellipse de centre A, le point B occupe un des foyers, le demi petit axe correspond au rayon RA du cercle C_{A} et le demi grand axe correspond à la distance A-B + RB, où RB est le rayon du cercle C_{B} ;
- Détermination de la position du point A (Xa, Ya) donnée par le centre de l'ellipse ;
- Détermination de la profondeur d'interaction Za en A qui est donnée par le demi grand axe de l'ellipse RA;
- Calcul du temps Ta en corrigeant les temps mesurés avec Za ;
- Détermination de la position du point B (Xb, Yb) qui est donnée par le foyer de l'ellipse ;
- Détermination de la profondeur d'interaction Zb en B qui est donnée par RB calculé à partir du demi grand axe de l'ellipse : par Distance (A - B) + RB ;
- Calcul du temps Tb en corrigeant les temps mesurés avec Zb ;
- Mesure de l'énergie totale Ea + Eb en intégrant les photons sur l'ensemble de la dite ellipse ;
- Mesure du barycentre de la distribution des photons dans l'ellipse ;
- Détermination du point initial d'interaction A ou B, ledit point initial est celui qui est le plus proche du barycentre ;
- Détermination de l'angle de déviation Compton α_{C} en reconstituant la position des deux interactions en A et en B.

13. Procédé de détermination des coordonnées spatio temporel (X, Y, Z, T) et de l'énergie E selon la revendication 12 lorsque les cercles C_{A} et C_{B} sont confondus et α_{C} > θ_{C}, **caractérisé en ce qu'**il peut comprend également des étapes suivantes :
- Ajustement de la distribution globale de lumière par une composition de deux cercles C_{A} et C_{B};
- Détermination des positions (Xa, Ya) et (Xb, Yb) des interactions respectives en A et en B, lesdites positions sont données par le centre de chaque cercle C_{A} et C_{B} ;
- Détermination de la profondeur des interactions Za et Zb, en déterminant le diamètre des cercles C_{A} et C_{B} ;
- Mesure de l'énergie totale Ea + Eb en intégrant les photons sur l'ensemble de ladite composition ;
- Détermination du barycentre de la distribution globale de lumière des photons dans la composition de deux cercles ;
- Détermination du point initial d'interaction A ou B, ledit point initial est celui qui est le plus proche du barycentre de la distribution globale de lumière ;
- Détermination de l'angle de déviation Compton α_{C} en reconstituant la position des deux interactions en A et en B.

14. Utilisation du système de détection selon l'une des revendications 1 à 11 dans le domaine notamment de l'astronomie de l'industrie nucléaire et du médical et de l'industrie pour détecter des contaminations radioactives.

## Patentansprüche

1. System zur Erkennung von Gammastrahlung vom Compton-Kamera-Typ, umfassend eine Gammastrahlungsquelle, eine schnelle Szintillator-Platte P1, deren Zeit zum Anstieg zum Lichtspitzenwert weniger als 1 ns beträgt, die eine Dicke aufweist, die größer als oder gleich 5 mm ist, mit einem Netz von segmentierten Photodetektoren (5) und einer dedizierten Mikroelektronik zum schnellen Lesen ausgestattet, wobei das System **dadurch gekennzeichnet ist, dass** es ein Modul zur Schätzung eines gültigen Compton-Ereignisses umfasst, das dazu in der Lage ist, die Raum-Zeit-Koordinaten (X, Y, Z, T) und die Energie E an mindestens zwei aufeinanderfolgenden Positionen eines Gammaphotons zu messen, wenn das Photon einer Compton-Ablenkung an einem ersten Punkt A zu einer Zeit TA unterzogen wird, bevor es an einem zweiten Punkt B zu einer Zeit TB absorbiert wird, indem Kreise von nicht gestreuten Photonen erkannt werden, die jeder Szintillationswechselwirkung entsprechen, wobei das Modul dazu fähig ist, diese Schätzung eines Ereignisses umzusetzen, indem es mindestens einen ersten und einen zweiten Extremwert in der Lichtverteilung im Inneren der Platte P1 identifiziert, wobei der zweite Extremwert auftritt, wenn die Differenz zwischen einer Zeit Ta der Ankunft von Photonen an einem ersten Punkt A und einer Zeit Tb der Ankunft von Photonen an einem zweiten Punkt B kleiner als das Dreifache eine Übertragungszeit Tt des Lichts in der Platte P1 ist, und dass das System dazu fähig ist, einerseits einen Kreis C_{A}, der den nicht gestreuten, emittierten Photonen durch die Compton-Ablenkung der Gammastrahlung am Punkt A entspricht, wobei der Durchmesser des Kreises C_{A} ermöglicht, Xa, Ya und Za zu messen, und andererseits einen Kreis C_{B}, der den nicht gestreuten, emittierten Photonen durch die vollständige Absorption des Gammaphotons am Punkt B entspricht, wobei der Durchmesser des Kreises C_{B} ermöglicht, Xb, Yb und Zb zu messen, zu bestimmen.

2. System zur Erkennung von Gammastrahlung vom Compton-Kamera-Typ, umfassend eine Gammastrahlungsquelle, zwei schnelle Szintillator-Platten (P1, P2), deren Zeit zum Anstieg zum Lichtspitzenwert weniger als 1 ns beträgt, wobei die Platte P1 feiner als die zweite Platte P2 ist, wobei die Dicke der Szintillator-Platte P1 derart ist, dass das Gammaphoton einer Compton-Ablenkung an einem Punkt A der Platte P1 unterzogen wird, wobei die zweite Platte P2 von der Platte P1 durch einen Abstand von mindestens 10 mm, vorzugsweise von mehr als der Dicke der dicksten Platte getrennt ist, ein Netz von segmentierten Photodetektoren und eine dedizierte Mikroelektronik zum schnellen Lesen, wobei das System **dadurch gekennzeichnet ist, dass** es ein Schätzungsmodul umfasst, das dazu in der Lage ist, die Raum-Zeit-Koordinaten (X, Y, Z, T) und die Energie E an mindestens zwei aufeinanderfolgenden Positionen eines Gammaphotons zu messen, wenn das Photon einer Compton-Ablenkung an einem ersten Punkt A unterzogen wird, bevor es an einem zweiten Punkt B absorbiert wird, indem Kreise von nicht gestreuten Photonen erkannt werden, die jeder Szintillationswechselwirkung entsprechen, wobei das Modul dazu fähig ist, auf der zweiten Platte P2 einen Koinzidenz-Trigger in einem Zeitfenster zu messen, das kleiner als die maximale Übertragungszeit des Lichts zwischen den Platten P1 und P2 ist, um die gültigen Compton-Ereignisse zu identifizieren, wobei dieser Koinzidenz-Trigger kleiner als 1 ns ist, und dass es dazu fähig ist, einerseits einen Kreis C_{A}, der den nicht gestreuten, emittierten Photonen durch die Compton-Ablenkung der Gammastrahlung am Punkt A entspricht, wobei der Durchmesser des Kreises C_{A} ermöglicht, Xa, Ya und Za zu messen, und andererseits einen Kreis C_{B}, der den nicht gestreuten, emittierten Photonen durch die vollständige Absorption des Gammaphotons am Punkt B entspricht, wobei der Durchmesser des Kreises C_{B} ermöglicht, Xb, Yb und Zb zu messen, zu bestimmen.

3. System zur Erkennung von Gammastrahlung vom Compton-Kamera-Typ nach Anspruch 1, **dadurch gekennzeichnet, dass** es eine einzige Szintillator-Platte P1 mit einer Dicke, die größer als oder gleich der durchschnittliche freie Weg des Gammastrahls in dem betreffenden Kristall ist, umfasst.

4. System zur Erkennung von Gammastrahlung vom Compton-Kamera-Typ nach Anspruch 3, **dadurch gekennzeichnet, dass** es zwei Netze von Photodetektoren umfasst, die jeweils auf einer Eintrittsfläche (1) und einer Austrittsfläche (3) der Szintillator-Platte P1 angeordnet sind, um die Präzision der Rekonstruktion der Compton-Bahn in der Szintillator-Platte P1 zu verbessern.

5. System zur Erkennung von Gammastrahlung vom Compton-Kamera-Typ nach Anspruch 4, **dadurch gekennzeichnet, dass** die Eintrittsfläche (1) und die Austrittsfläche (3) der Szintillator-Platte (P1), die an das Netz von Photodetektoren (5) gekoppelt sind, poliert sind und die Kopplung zwischen den Flächen und dem Netz von Photodetektoren durch ein Indexmedium n, das kleiner als 1,5 ist, umgesetzt ist, um einen Gesamtreflexionswinkel zu erreichen.

6. Erkennungssystem nach Anspruch 1, **dadurch gekennzeichnet, dass** seitliche Flächen und eine Eintrittsfläche (1) der Platte P1, die nicht an ein Netz von Photodetektoren (5) gekoppelt sind, unpoliert sind, wobei die Flächen derart behandelt sind, dass bei der Absorption von einfallenden Photonen oder der diffusen Reflexion von Photonen ein Maximum vorliegt.

7. Erkennungssystem nach Anspruch 6, **dadurch gekennzeichnet, dass** die Eintrittsfläche (1) der Platte P1, die nicht an ein Netz von Photodetektoren gekoppelt ist, schwarz angestrichen ist, um die Reflexion auf der Fläche zu begrenzen.

8. Erkennungssystem nach Anspruch 1, **dadurch gekennzeichnet, dass** seitliche Flächen und eine Eintrittsfläche (1) der Platte (P1), die nicht an ein Netz von Photodetektoren (5) gekoppelt sind, durch einen weißen Reflektor mit einem Luftspalt mit der Platte P1 umhüllt sind, so dass sie reflektierend und streuend sind.

9. System nach einem der vorhergehenden Ansprüche, **dadurch gekennzeichnet, dass** die Messung der Energie auf herkömmliche Weise vorgenommen wird, indem ein Maximum von emittierten Photonen auf mindestens einer von zwei Platten durch diffuse Reflexion aufgenommen wird.

10. System nach einem der vorhergehenden Ansprüche, **dadurch gekennzeichnet, dass** das fragmentierte Netz von Photodetektoren (5) vom analogen SI-PM-Typ, der mit einem analogen ASIC assoziiert ist, oder vom digitalen SI-PM-Typ ist.

11. System nach einem der vorhergehenden Ansprüche, **dadurch gekennzeichnet, dass** die Szintillator-Platten P1 und P2 vom Lutetiumsilicat- und/oder Lanthanhalogenid-Typ sind.

12. Verfahren zur Bestimmung von Raum-Zeit-Koordinaten (X, Y, Z, T) und der Energie E an mindestens zwei aufeinanderfolgenden Positionen eines Gammaphotons, das einem Compton-Effekt unterzogen wurde, das in einem System nach einem der Ansprüche 1 bis 11 ausgeführt wird, umfassend die folgenden Schritte:
- Erkennen einer Zeit Ta der Ankunft von nicht gestreuten, emittierten Photonen durch den Compton-Effekt an einem ersten Punkt A;
- Erkennen einer Zeit Tb der Ankunft von nicht gestreuten, emittierten Photonen an einem zweiten Punkt B durch die vollständige Absorption des Gammaphotons;
- Bestimmen eines Kreises C_{A}, der den nicht gestreuten, emittierten Photonen durch die Compton-Ablenkung der Gammastrahlung am Punkt A entspricht, wobei der Durchmesser des Kreises C_{A} ermöglicht, Xa, Ya und Za zu messen;
- Bestimmen eines Kreises C_{B}, der den nicht gestreuten, emittierten Photonen durch die vollständige Absorption des Gammaphotons am Punkt B entspricht, wobei der Durchmesser des Kreises C_{B} ermöglicht, Xb, Yb und Zb zu messen;
wobei, wenn die emittierten Photonen während des Compton-Effekts an A und der vollständigen Absorption an B in demselben Lichtkegel von nicht gestreuten, emittierten Photonen an A bleiben, der Winkel α_{c} < θ_{c}, wobei α_{c} der Compton-Effekt ist und θ_{c} der kritische Gesamtreflexionswinkel ist, und der Kreis C_{B} in diesem ersten Fall in dem Kreis C_{A} enthalten ist, das Verfahren weiterhin folgende Schritte umfasst:
- Berechnen von Durchmessern der Kreise C_{A} und C_{B}, um (Xa, Ya, Za) und (Xb, Yb, Zb) zu messen;
- Aufzählen von Anzahlen von Photonen in den registrierten Kreisen C_{A} und C_{B};
- Definieren der Energie eines Gammaphotons, wobei die Energien Ea und Eb proportional zur Anzahl von Photonen, die im Inneren der Kreise C_{A} und C_{B} gezählt werden, sind; oder
wobei, wenn das Photon, das einem Compton-Effekt unterzogen wurde, aus dem Lichtkegel austritt, α_{c} > θ_{c}, der Abstand zwischen den Punkten A und B groß ist und die Kreise C_{A} und C_{B} in dieser zweiten Konfiguration verschieden sind, das Verfahren weiterhin umfasst:
- Bestimmen eines ersten Ereignisses A, das der größten Energie entspricht;
- Messen von Koordinaten (Xa, Ya, Za, Ta) des Ereignisses an A und seiner Energie Ea;
- Bestimmen eines zweiten Ereignisses B, das der kleinsten Energie entspricht;
- Messen von Koordinaten des Ereignisses (Xb, Yb, Zb, Tb) und seiner Energie Eb;
- Messen der Anfangsenergie des Gammaphotons, die gleich der Summe der Energien Ea + Eb ist;
- Bestimmen eines Winkels des Compton-Effekts, indem die Position von zwei Wechselwirkungen rekonstruiert wird;
- Ableiten der Richtung der Ankunft des Gammaphotons aus der Position des Punkts A (Xa, Ya, Za), der Position des Punkts B (Xb, Yb, Zb) und der Energien Ea und Eb; oder
wobei, wenn das Photon, das einem Compton-Effekt unterzogen wurde, aus dem Lichtkegel austritt, α_{c} > θ_{c}, der Abstand zwischen den Punkten A und B klein ist und die Kreise C_{A} und C_{B} in diesem dritten Fall zusammengenommen sind, das Verfahren die folgenden Schritte umfassen kann:
- Anpassen der Lichtverteilung durch eine Mittelellipse A, wobei der Punkt B einen der Brennpunkte belegt, wobei die kleine Halbachse dem Radius RA des Kreises C_{A} entspricht und die große Halbachse dem Abstand A-B + RB entspricht, wobei RB der Radius des Kreises C_{B} ist;
- Bestimmen der Position des Punkts A (Xa, Ya), die durch die Mitte der Ellipse gegeben ist;
- Bestimmen der Tiefe der Wechselwirkung Za an A, die durch die große Halbachse der Ellipse RA gegeben ist;
- Berechnen der Zeit Ta, indem die gemessenen Zeiten mit Za korrigiert werden;
- Bestimmen der Position des Punkts B (Xb, Yb), die durch den Brennpunkt der Ellipse gegeben ist;
- Bestimmen der Tiefe der Wechselwirkung Zb an B, die durch RB gegeben ist, der aus der großen Halbachse der Ellipse berechnet wird: durch den Abstand (A - B) + RB;
- Berechnen der Zeit Tb, indem die gemessenen Zeiten mit Zb korrigiert werden;
- Messen der Gesamtenergie Ea + Eb, indem die Photonen auf der Gesamtheit der Ellipse integriert werden;
- Messen des Schwerpunkts der Verteilung von Photonen in der Ellipse;
- Bestimmen des Anfangspunkts der Wechselwirkung A oder B, wobei der Anfangspunkt derjenige ist, der dem Schwerpunkt am nächsten ist;
- Bestimmen des Winkels des Compton-Effekts α_{c}, indem die Position von zwei Wechselwirkungen an A und an B rekonstruiert wird.

13. Verfahren zur Bestimmung von Raum-Zeit-Koordinaten (X, Y, Z, T) und der Energie E nach Anspruch 12, wenn die Kreise C_{A} und C_{B} zusammengenommen sind und α_{c} > θ_{c}, **dadurch gekennzeichnet, dass** es außerdem folgende Schritte umfassen kann:
- Anpassen der gesamten Lichtverteilung durch eine Zusammensetzung der zwei Kreise C_{A} und C_{B};
- Bestimmen der Positionen (Xa, Ya) und (Xb, Yb) von jeweiligen Wechselwirkungen an A und an B, wobei die Positionen durch die Mitte jedes Kreises C_{A} und C_{B} gegeben sind;
- Bestimmen der Tiefe von Wechselwirkungen Za und Zb, indem der Durchmesser der Kreise C_{A} und C_{B} bestimmt wird;
- Messen der Gesamtenergie Ea + Eb, indem die Photonen auf der Gesamtheit der Zusammensetzung integriert werden;
- Bestimmen des Schwerpunkts der gesamten Lichtverteilung von Photonen in der Zusammensetzung der zwei Kreise;
- Bestimmen des Anfangspunkts der Wechselwirkung A oder B, wobei der Anfangspunkt derjenige ist, der dem Schwerpunkt der gesamten Lichtverteilung am nächsten ist;
- Bestimmen des Winkels des Compton-Effekts α_{c}, indem die Position von zwei Wechselwirkungen an A und an B rekonstruiert wird.

14. Verwendung des Erkennungssystems nach einem der Ansprüche 1 bis 11 insbesondere im Gebiet der Astronomie, der Nuklearindustrie und der Medizin und der Industrie zum Erkennen von radioaktiven Kontaminationen.

## Claims

1. System for the detection of gamma radiation, of the Compton camera type, comprising a gamma radiation source, a fast scintillator plate P1 of which the rise time to peak light is less than 1 ns, having a thickness greater than or equal to 5 mm, equipped with an array of segmented photodetectors (5) and dedicated fast-reading microelectronic means, said system being **characterised in that** it comprises a module for estimating a valid Compton event capable of measuring the spatiotemporal coordinates (X, Y, Z, T) and energy E in at least two successive positions of a gamma photon when said photon undergoes Compton deviation at a first point A at a time TA before being absorbed at a second point B at a time TB, by recognising circles of unscattered photons corresponding to each scintillation interaction, said module being capable of carrying out this estimation of an event by identifying at least one first and one second extremum in the distribution of light within said plate P1, said second extremum appearing when the difference between a time Ta of arrival of photons at a first point A and a time Tb of arrival of photons at a second point B is less than three times a time of transfer Tt of light in said plate P1, and **in that** said system is capable of determining, on the one hand, a circle C_{A} corresponding to the unscattered photons emitted by Compton deviation of gamma radiation at point A, the diameter of the circle C_{A} making it possible to measure Xa, Ya and Za, and on the other hand a circle C_{B} corresponding to the unscattered photons emitted by total absorption of the gamma photon at point B, the diameter of the circle C_{B} making it possible to measure Xb, Yb and Zb.

2. System for the detection of gamma radiation, of the Compton camera type, comprising a gamma radiation source, two fast scintillator plates (P1, P2) of which the rise time to peak light is less than 1 ns, plate P1 being thinner than the second plate P2, the thickness of scintillator plate P1 being such that the gamma photon undergoes Compton deviation at a point A of said plate P1, said second plate P2 being separated from plate P1 by a distance 'D' of at least 10 mm, preferably greater than the thickness of the thicker plate, an array of segmented photodetectors and dedicated fast-reading microelectronic means, said system being **characterised in that** it comprises an estimating module capable of measuring the spatiotemporal coordinates (X, Y, Z, T) and energy E in at least two successive positions of a gamma photon when said photon undergoes Compton deviation at a first point A before being absorbed at a second point B, by recognising circles of unscattered photons corresponding to each scintillation interaction, said module being capable of measuring on said second plate P2 a coincidence trigger in a time window shorter than the maximum time of transfer of light between the plates P1 and P2 in order to identify valid Compton events, this coincidence trigger being less than 1 ns, and **in that** it is capable of determining on the one hand a circle C_{A} corresponding to the unscattered photons emitted by Compton deviation of gamma radiation at point A, the diameter of circle C_{A} making it possible to measure Xa, Ya and Za, and on the other hand a circle C_{B} corresponding to the unscattered photons emitted by total absorption of the gamma photon at point B, the diameter of circle C_{B} making it possible to measure Xb, Yb and Zb.

3. System for the detection of gamma radiation of the Compton camera type according to claim 1, **characterised in that** it comprises a single scintillator plate P1 having a thickness greater than or equal to the average free path of the gamma ray in the crystal in question.

4. System for the detection of gamma radiation of the Compton camera type according to claim 3, **characterised in that** it comprises two arrays of photodetectors disposed on an input face (1) and an output face (3) of said scintillator plate P1 respectively in order to improve the precision of reconstitution of the Compton path in the scintillator plate P1.

5. System for the detection of gamma radiation of the Compton camera type according to claim 4 in which the input face (1) and the output face (3) of the scintillator plate (P1), being coupled to the array of photodetectors (5), are polished, and coupling between said faces and the array of photodetectors is provided by having a media having an index n lower than 1.5, in order to form an angle of total reflection.

6. Detection system according to claim 1, **characterised in that** lateral faces and an input face (1) of said plate P1, not being coupled to an array of photodetectors (5), are rough, said faces are treated so that the absorption of incident photons or diffuse reflection of photons is maximal there.

7. Detection system according to claim 6 in which the input face (1) of the plate P1 not coupled to an array of photodetectors is painted black in order to limit reflection on said face.

8. Detection system according to claim 1, **characterised in that** lateral faces and an input face (1) of said plate (P1), not being coupled to an array of photodetectors (5), are coated with a white reflector with an air gap from the plate P1, so that they are reflective and scattering.

9. System according to one of the preceding claims, **characterised in that** energy measurement is done in a conventional manner by collecting, by diffuse reflection, a maximum of photons emitted on at least one of the two plates.

10. Detection system according to one of the preceding claims in which the fragmented photodetector array (5) is of the analogue SI-PM type associated with an analogue ASIC or an ASIC of the digital SI-PM type.

11. Detection system according to one of the preceding claims, **characterised in that** the scintillator plates P1 and P2 are of the lutetium silicate and/or lanthanum halide type.

12. Method for determining the spatiotemporal coordinates (X, Y, Z, T) and energy E in at least two successive positions of a gamma photon having undergone Compton scattering, carried out in a system according to one of claims 1 to 11, comprising the following steps:
- detection of a time Ta of arrival of unscattered photons emitted by Compton scattering at a first point A;
- detection of a time Tb of arrival of unscattered photons emitted at a second point B by total absorption of the gamma photon;
- Determination of a circle C_{A} corresponding to the unscattered photons emitted by Compton deviation of gamma radiation at point A, the diameter of the circle C_{A} making it possible to measure Xa, Ya and Za;
- Determination of a circle C_{B} corresponding to the unscattered photons emitted by total absorption of the gamma photon at point B, the diameter of the circle C_{B} making it possible to measure Xb, Yb and Zb;
when the photons emitted during Compton scattering at A and total absorption at B remain in the same light cone of unscattered photons emitted at A, the angle *α* _{c} < *θ* _{c} ,
where *α* _{c} is the Compton deviation and *θ* _{c} is the critical angle of total reflection and circle C_{B} is included in circle C_{A}, in this first case, the method further comprises the following steps:
- Calculation of the diameters of said circles C_{A} and C_{B} in order to measure (Xa, Ya, Za) and (Xb, Yb, Zb);
- Enumeration of the numbers of photons within circles C_{A} and C_{B} one inside the other;
- Definition of the energy of a gamma photon, said energies Ea and Eb being proportional to the number of photons counted within said circles C_{A} and C_{B}; or
when the photon that has undergone Compton deviation exits from the light cone, *α* _{c} > *θ* _{c} , the distance between points A and B is great and the circles C_{A} and C_{B} are separate in this second configuration, the method further comprises:
- Determination of a first event A corresponding to the highest energy;
- Measurement of the coordinates (Xa, Ya, Za, Ta) of said event at A and its energy Ea;
- Determination of a second event B corresponding to the lowest energy;
- Measurement of the coordinates of the event (Xb, Yb, Zb, Tb) and its energy Eb;
- Measurement of the initial energy of the gamma photon equivalent to the sum of energies Ea + Eb;
- Determination of an angle of Compton deviation by reconstituting the position of the two interactions;
- Deduction of the direction of arrival of the gamma photon, on the basis of the position of point A (Xa, Ya, Za), the position of point B (Xb, Yb, Zb) and the energies Ea and Eb; or
when the photon that has undergone Compton deviation exits from the light cone, *α* _{c} > *θ* _{c}, the distance between points A and B is small and the circles C_{A} and C_{B} coincide, in this third case, the method may comprise the following steps:
- Adjustment of the distribution of light by an ellipse of centre A, point B occupies one of the foci, the semi-minor axis corresponds to the radius RA of circle C_{A} and the semi-major axis corresponds to the distance A-B + RB, where RB is the radius of circle C_{B};
- Determination of the position of point A (Xa, Ya) given by the centre of the ellipse;
- Determination of the depth of interaction Za at A which is given by the semi-major axis of the ellipse RA;
- Calculation of the time Ta by correcting the times measured with Za;
- Determination of the position of point B (Xb, Yb) which is given by the focus of the ellipse;
- Determination of the depth of interaction Zb at B which is given by RB calculated from the semi-major axis of the ellipse divided by Distance (A - B) + RB;
- Calculation of the time Tb by correcting the times measured with Zb;
- Measurement of the total energy Ea + Eb by integrating the photons on the whole of said ellipse;
- Measurement of the barycentre of distribution of photons in the ellipse;
- Determination of the initial point of interaction A or B, said initial point is the one which is closest to the barycentre;
- Determination of the angle of Compton deviation *α* _{c} by reconstituting the position of the two interactions at A and B.

13. Method for determining the spatiotemporal coordinates (X, Y, Z, T) and energy E according to claim 12 when the circles C_{A} and C_{B} coincide and *α* _{c} > *θ* _{c} , **characterised in that** it may also comprise the following steps:
- Adjustment of the total distribution of light by a composition of two circles C_{A} and C_{B};
- Determination of the positions (Xa, Ya) and (Xb, Yb) of the respective interactions at A and at B, said positions are given by the centre of each circle C_{A} and C_{B};
- Determination of the depth of interactions Za and Zb, by determining the diameter of the circles C_{A} and C_{B};
- Measurement of the total energy Ea + Eb by integrating the photons on the whole of said composition;
- Determination of the barycentre of total distribution of light of the photons in the composition of two circles;
- Determination of the initial point of interaction A or B, said initial point is the one which is closest to the barycentre of total distribution of light;
- Determination of the angle of Compton deviation *α* _{c} by reconstituting the position of the two interactions at A and at B.

14. Use of the detection system according to one of claims 1 to 11 in the field in particular of astronomy, the nuclear industry and medicine and the industry for detecting radioactive contamination.
